# EUROPÄISCHE PATENTSCHRIFT

(11) **EP 2 334 346 B1**
(45) Veröffentlichungstag und Bekanntmachung des Hinweises auf die Patenterteilung: **02.11.2016**
(21) Anmeldenummer: 09782919.6
(22) Anmeldetag: 11.09.2009
(51) Int. Cl.: A61L 27/24, A61L 27/34, A61L 27/44, A61L 27/46, A61L 27/52

(54) **KOMPOSITMATERIALIEN AUS EINER MIT SILIKAT UND CALCIUMPHOSPHATPHASEN MINERALISIERTEN KOLLAGENMATRIX, VERFAHREN ZU DEREN HERSTELLUNG UND DEREN VERWENDUNG**
COMPOSITE MATERIAL CONSISTING OF A COLLAGEN MATRIX MINERALISED WITH SILICATE AND CALCIUM PHOSPHATE PHASES, METHOD FOR THE PRODUCTION AND USE THEREOF
MATÉRIAUX COMPOSITES À MATRICE COLLAGÉNIQUE MINÉRALISÉE AVEC DU SILICATE ET DES PHASES DE PHOSPHATE DE CALCIUM, LEUR PROCÉDÉ DE FABRICATION ET LEUR UTILISATION

(30) Priorität: 11.09.2008 DE 102008047405
(43) Veröffentlichungstag der Anmeldung: 22.06.2011
(73) Patentinhaber: Technische Universität Dresden, 01069 Dresden (DE)
(72) Erfinder: HEINEMANN, Sascha, 01069 Dresden (DE); WORCH, Hartmut, 01187 Dresden (DE); HANKE, Thomas, 13187 Berlin (DE)
(74) Vertreter: Kailuweit & Uhlemann Patentanwälte Partnerschaft mbB
(86) Internationale Anmeldenummer: PCT/EP2009/061809
(87) Internationale Veröffentlichungsnummer: WO 2010/029150

(56) Entgegenhaltungen:
- WO-A1-2005/099785
- WO-A1-2008/023025
- DE-A1- 19 962 090
- DE-A1-102004 012 411
- US-A1- 2008 147 197

## Beschreibung

Die Erfindung betrifft neue Kompositmaterialien auf der Basis einer mit Silikat und Calciumphosphatphasen mineralisierten Kollagenmatrix, Verfahren zu deren Herstellung sowie deren Verwendung als vielfältig gestaltbares Implantatmaterial, biologisierende Beschichtung oder Wirkstoffträger.

Die Entwicklung neuer Biomaterialien wird hauptsächlich angetrieben durch das Bestreben, ausgereifte Gewebe- und Knochenimplantate für medizinische Anwendungen herzustellen. An diese werden jedoch je nach Implantationsort und Funktion spezielle Ansprüche bezüglich der Bioaktivität, Biokompatibilität und der mechanischen Festigkeit gestellt. Diese Anforderungen können von einkomponentigen Materialien in der Regel kaum erfüllt werden. Biologische Komponenten, die aus funktionellen Gründen meist vorausgesetzt werden, weisen zwar oftmals eine gute Biokompatibilität auf, sind dabei jedoch nicht in der Lage, größeren mechanischen Belastungen standzuhalten. Aus diesem Grund wird zunehmend an Strategien zur Verstärkung solcher Materialien gearbeitet. Beispielsweise wäre dies durch die Kombination mit weiteren, anorganisch-nichtmetallischen Komponenten erreichbar, die entsprechende Lasten aufnehmen können, die aber ihrerseits ausreichend biokompatibel sein müssen.

Unter Bioaktivität versteht man die Eigenschaft eines Materials in Umgebung von (simulierter) Körperflüssigkeit die Bildung einer Apatitschicht an seiner Oberfläche zu fördern. Besonders im Bereich der Knochenersatzmaterialien ist eine möglichst hohe Bioaktivität notwendig, um eine kraftschlüssige Verbindung zwischen Implantat und Empfängergewebe herzustellen. Materialien auf der Basis von Kollagen sind für biomedizinische Anwendungen von besonders großem Interesse. Als körpereigenes Strukturprotein kommt Kollagen ubiquitär in allen tierischen Vielzellern vor und ist mit etwa einem Drittel der gesamten Proteinmasse deren häufigstes Protein. Es ist nahezu untoxisch, bioresorbierbar und kaum immunogen, wodurch sich eine exzellente Biokompatibilität ergibt. Als Ausgangsmaterial für industrielle Anwendungen wird meist Kollagen vom Typ I verwendet, das zum Beispiel aus Sehnen, Knorpel und Häuten von Rindern, Kälbern und Schweinen gewonnen werden kann. Mittlerweile wurden zahlreiche Produkte auf der Basis von Kollagen entwickelt und in vielen Bereichen der Kosmetik und Medizin etabliert. Weitergehende Anwendungen verhindern jedoch oftmals die begrenzten mechanischen Eigenschaften reiner Kollagenprodukte.

Im Tierreich hat die Evolution diesen Mangel eindrucksvoll beseitigt, indem lasttragende Elemente eines Organismus aus Materialverbunden aufgebaut werden. Die vorliegende Anmeldung vereint die Grundprinzipien der natürlichen Vorbilder, nämlich Spikulen mariner Glasschwämme und Säugetierknochen.

Durch die Erfinder konnte nachgewiesen werden, dass bereits die 600 Millionen Jahre alten marinen Glasschwämmen ein Beispiel für natürlich vorkommende Kompositmaterialien liefern [Ehrlich, H., Hanke, T., Simon, P., Goebel, C., Heinemann, S., Born, R., Worch, H.: Demineralization of natural silica based biomaterials: new strategy for the isolation of organic frameworks. In: BIOmaterialien 6 (2005), Nr. 4, S. 297-302; Ehrlich, H., Ereskovskii, A. V., Drozdov, A. L., Krylova, D. D., Hanke, T., Meissner, H., Heinemann, S., Worch, H.: A Modern Approach to Demineralization of Spicules in Glass Sponges (Porifera: Hexactinellida) for the Purpose of Extraction and Examination of the Protein Matrix. In: Russ Marin Biol 32 (2006), Nr. 3, S. 186-93]. Moderne Analysemethoden offenbarten, dass die Spikulen des marinen Glasschwammes *Hyalonema sieboldi* aus einer silikatisierten Kollagenmatrix bestehen, woraus sich deren besondere mechanische Eigenschaften ergaben.

Säugetierknochen muss neben anderen Funktionen als Stützelement im Körper vielseitigen mechanischen Belastungen standhalten [Wintermantel, E., Ha, S.-W.: Biokompatible Werkstoffe und Bauweisen. Springer Verlag, 1998]. Trotz einer hohen Festigkeit (Druckspannungen) muss er jedoch auch eine gewisse Elastizität aufweisen, damit er auch unter ungünstigen Belastungszuständen (Hebelkräfte) nicht spröde bricht. Diesen Anforderungen wird der Knochen durch seinen hierarchischen Aufbau und ganz entscheidend auch durch seine stoffliche Zusammensetzung auf molekularer Ebene gerecht. Die organische Knochenmatrix, die ca. 30 % des Knochens ausmacht, besteht zu ca. 95 % aus Kollagen, das den Part der elastischen Komponente zur Aufnahme von Zug- und Biegespannungen einnimmt. Im Zuge eines Mineralisationsprozesses wird zwischen den Kollagenfibrillen mineralisches Calciumphosphat (vor allem Hydroxylapatit) in Gestalt etwa zwei bis vier Nanometer dicker kristalliner Plättchen eingelagert [Kapitel Forschung aktuell. In:MaxPlankForschung (Hrsg.): Knochen auf den Zahn gefühlt. Bd. 1. 2005, S. 10-1].

Je nach Grad der Mineralisation (beim Knochen ca. 65 %) wird das flexible Grundgerüst aus Kollagen mechanisch verstärkt, wodurch sich die Festigkeit, vor allem unter Druckbelastung, enorm erhöht. Aufbauend auf derartigen Erkenntnissen wird seit einiger Zeit intensiv an Möglichkeiten zur Nachahmung natürlich vorkommender Verbundmaterialien - vor allem des Knochens - gearbeitet, was jedoch nur in sehr begrenztem Maße gelungen ist.

In der Patentanmeldung WO 2008/023025, PCT/EP2007/058694 beschreiben die Erfinder eine Methode, um Hybridmaterialien basierend auf einer silikatisierten Kollagenmatrix herzustellen. Sie basiert auf einem Sol-Gel-Prozess, in dem Kieselsäuremoleküle unter bestimmten Bedingungen zu Silikat-Nanopartikeln polymerisieren. Wenn dieser Prozess in Gegenwart von Kollagenfibrillen durchgeführt wird, findet die Polymerisation vorzugsweise an den Fibrillen statt. Bei geeigneter Zusammensetzung lässt sich auf diese Weise ein Hybrid-Hydrogel herstellen. Dieses kann unter geeigneten Bedingungen getrocknet und so in ein Hybrid-Xerogel überführt werden. Da der Feststoffanteil des Hydrogels prozessbedingt jedoch sehr gering ist, lassen sich nur kleine Abmessungen eines monolithischen Materials herstellen. Bei dem Zweistoffsystem Silikat-Kollagen ist der Zusammensetzungsbereich, in dem monolithische Xerogele hergestellt werden können, begrenzt. Das resultierende Gefüge lässt sich ausschließlich über die jeweiligen Komponentenanteile des Silikats und des Kollagens beeinflussen. Darum lassen sich auch die Materialeigenschaften, und dabei vor allem die mechanischen Eigenschaften, nur in einem engen Bereich variieren.

Aus der deutschen Patentanmeldung DE 10 2004 012 411 A1 sind Kompositmaterialien auf der Basis von Polykieselsäuren und Verfahren zu deren Herstellung bekannt, die Polykieselsäure, ein organisches Polymer im Anteil von 0,1 bis 20 Masse- %, mindestens eine Calciumphosphatphase mit einem Anteil von mehr als 15 Masse- % und wahlweise ein anwendungsspezifisches Additiv enthalten. Um zu den offenbarten Kompositmaterialien gelangen, werden die einzelnen Bestandteile des Kompositmaterials in Abhängigkeit von ihren chemischen und physikalischen Eigenschaften aufeinanderfolgend oder kombinationsweise zusammengegeben. Anschließend wird die Mischung homogenisiert, in die gewünschte Form gebracht und dann bei Temperaturen von 100 °C und mehr getrocknet.

Die deutsche Patentanmeldung DE 10 2006 014 522 A1 offenbart ein Verfahren zur Herstellung eines Knochenersatzmaterials, bei dem ein als Lösungsmittel Wasser und/oder Alkohol aufweisendes Sol einer oxidischen Verbindung mit darin homogen verteilten Calciumphosphatphasen in eine unterhalb des Gefrierpunktes des verwendeten Lösungsmittels abgekühlte Gefrierform gegossen wird. Das Lösungsmittel wird unter Gelbildung gefroren und anschließend das gefrorene Lösungsmittel durch Gefriertrocknung entzogen, wodurch ein offenporiges Gelstützgerüst mit den eingebetteten Calciumphosphatphasen entsteht.

In der deutschen Patentanmeldung DE 198 11 600 A1 wird ein biokompatibles Kompositmaterial beschrieben, das ein anorganisches Gel und als bioaktive Komponente ein oder mehrere homogen eingebettete Skleroproteine oder deren Hydrolyseprodukte und/oder Glykosaminoglykane enthält. Durch Zusatz von Calciumsalzen, Phosphaten oder basischen Calciumphosphat-Suspensionen kann die Bioaktivität des Komposits erhöht werden.

Aus der deutschen Patentanmeldung DE 199 62 090 A1 sind Formkörper in der Geometrie von Knochen oder Knochenteilen bekannt, die aus Kollagen oder mineralisiertem Kollagen in Form eines dichten Netzwerks von Kollagenfibrillen ohne oder mit zusätzlich einer Matrix aus Calciumphosphatzement, in der die Kollagenfibrillen eingebettet sind, gebildet ist und die über ein Gefriertrocknungsverfahren hergestellt werden.

Viele weitere aus dem Stand der Technik bekannte Verfahren bedienen sich des Sol-Gel-Verfahrens. Diese werden dabei in der Regel nur als Vorstufe eingesetzt und das erhaltene Material wird im Anschluss gemahlen, gepresst und thermisch behandelt. Durch die hohen Temperaturen von meist mehreren hundert Grad Celsius sintert das Pulver dann zu einem Monolith zusammen. Organische Bestandteile werden bei diesen Bedingungen zerstört.

Aufgabe der Erfindung ist es, ein Stoffsystem zu entwickeln, das basierend auf den Ausgangskomponenten Silikat und Kollagen weitere Variationsmöglichkeiten eröffnet, mithilfe derer der Feststoffgehalt des während des Herstellungsverfahrens erhaltenen Hydrogels erhöht werden soll, eine hohe Bioaktivität des erhaltenen Kompositmaterials erzielt werden soll und das Gefüge und damit die mechanischen Eigenschaften des Kompositmaterials in einem weiten Bereich eingestellt werden können.

Eine weitere Aufgabe besteht darin, kompakte monolithische Stoffsysteme auch ohne thermische Nachbehandlung direkt mittels eines Sol-Gel-Prozesses herzustellen und so vorteilhaft zum einen schonende Verfahren zur Verfügung zu stellen, bei denen das enthaltene Kollagen nicht thermisch zerstört wird, sondern seine Faserstruktur behält und somit das Kompositmaterial verstärkt, und zum anderen den mit der Herstellung der Kompositmaterialien verbundenen Energieaufwand niedrig zu halten.

Erfindungsgemäß wird die Aufgabe gelöst durch ein Kompositmaterial nach Anspruch 1, umfassend eine mit Silikat und einer Calciumphosphatphase mineralisierte Kollagenmatrix.

Die Kollagenkomponente des erfindungsgemäßen Kompositmaterials kann dabei rekombinantes Kollagen, Kollagen aus Eumetazoa (d.h. Gewebetiere, darunter Nesseltiere und Bilateria), Schwammkollagen der Klassen Demospongia (Hornschwämme) oder Calcarea (Kalkschwämme), ein synthetisches Kollagenanalogon, ein Kollagenderivat oder eine Mischung dieser Kollagene sein.

Kollagenanaloga sind synthetisch hergestellte Polypeptidketten, deren Primärsequenz so gestaltet ist, dass sie bestimmte Eigenschaften der nativen Kollagenmonomere simulieren können, z.B. Tripelhelixbildung, Fibrillogenese, Gelbildung. Rekombinante Kollagene sind solche, deren Primärsequenz identisch ist mit der von Kollagen Typ I. Sie werden mithilfe genetisch manipulierter Mikroorganismen hergestellt und gegebenenfalls nachbehandelt. Kollagenderivate sind Verbindungen, die sich von der Grundverbindung Kollagen formal ableiten und aus ihr herstellen lassen.

Der neben Kollagen zweite Hauptbestandteil von Knochen ist Calciumphosphat in der Form von Hydroxylapatit. Durch die Einführung von Hydroxylapatit und anderen Calciumphosphatphasen in das erfindungsgemäße Kompositmaterial als zusätzliche anorganische Komponente, die in pulvriger Form in das System eingebracht wird, erhöhen sich der Feststoffanteil und die Bioaktivität des erfindungsgemäßen Kompositmaterials ganz erheblich. Dies wird beispielsweise demonstriert durch die Beschleunigung der Ausbildung einer Hydroxylapatitschicht *in vitro* in simulierter Körperflüssigkeit (Simulated body fluid, SBF).

Je nach Zusammensetzung der Calciumphosphatphase können dabei unterschiedliche Effekte erzielt werden. Das Gefüge wird ebenfalls beeinflusst, was sich auf die mechanischen Eigenschaften auswirkt. Einsetzbar sind alle Calciumphosphatverbindungen, die eine homogene Mischung mit der Silikatlösung und der Kollagensuspension erlauben. Vorzugsweise finden dabei solche Calciumphosphatphasen Verwendung, die in *vivo* in Hydroxylapatit umgewandelt werden. In einer bevorzugten Ausführungsform wird als Calciumphosphatphase, Hydroxylapatit (Ca₁₀(PO₄)₆(OH)₂), Tricalciumphosphat (Ca₃PO₄), amorphes Calciumphosphat (Caₓ(PO₄)_{y}·nH₂O), Octacalciumphosphat (Ca₈H₂(PO₄)₆·5H₂O) oder Brushit (CaHPO₄·2H₂O) oder Mischungen dieser Calciumphosphatphasen untereinander wie beispielsweise Calciumphosphatzement (eine Mischung aus Ca₃(PO₄)₂, CaCO₃, Ca(HCO₃) und Ca₁₀(PO₄)₆(OH)₂) oder Mischungen von bzw. mit Calcium- und Phosphatsalzen wie beispielsweise CaCl₂ und/oder Na₂HPO₄ genutzt. Die Calciumphosphatphase kann dabei zusätzlich mit Ionen wie Fluorid, Silber, Magnesium oder Carbonat dotiert sein.

In einer besonders bevorzugten Ausführungsform wird als Calciumphosphatphase nanoskopischer Hydroxylapatit (Ca₁₀(PO₄)₆(OH)₂) eingesetzt, da sich dieser besonders homogen einbringen lässt. Eine homogene Verteilung der Calciumphosphatphase im Material bewirkt besonders gut reproduzierbare Materialeigenschaften. Nanoskopischer Hydroxylapatit besteht im wesentlichen aus Hydroxylapatit- Nanopartikeln, die vorzugsweise eine Größe von 10 bis 500 nm, bevorzugt eine Größe von weniger als 100 nm aufweisen.

Kollagenfibrillen sind aufgebaut aus einzelnen Tropokollagenmolekülen, in denen sich je drei Peptidketten zu einer Tripelhelix von etwa 300 nm Länge zusammenlagern. Während der Fibrillogenese lagern sich Tropokollagenmoleküle zu größeren Kollagenfibrillen zusammen. Durch Bündelung von Kollagenfibrillen durch kovalente Quervernetzungen bilden sich wiederum Kollagenfasern.

Als Kollagenkomponente kann jegliches Kollagen eingesetzt werden, d.h. Kollagen jeglicher Form (z.B. fibrilläres Kollagen, netzbildendes Kollagen, faserbildendes Kollagen, Tropokollagen, teilweise oder vollständig fibrilliertes Kollagen, teilweise oder vollständig (irreversibel) denaturiertes Kollagen, Kollagenfasern oder größere Aggregate usw.) und jeglichen Typs (z.B. Typ I, II, III, IV, V, VI, VII, VIII, IX, X, XI, XII, XIII, XIV, XV, XVI, XVII, XVIII, XIX, XX, XXI, XXII, XXIII, XXIV, XXV, XXVI).

In dem erfindungsgemäßen Kompositmaterial kommt Kollagen in Form von Kollagenfibrillen oder von Kollagenfasern zum Einsatz, da dieses bezogen auf das reine Silikat vorteilhaft zur deutlichen Erhöhung der mechanischen Belastbarkeit führt.

In einer besonders bevorzugten Ausführungsform wird die organische Komponente Kollagen zusätzlich biochemisch modifiziert. Die Modifikation der Kollagenkomponente beruht dabei auf einer gezielten Beeinflussung der Fibrillengeometrie (Durchmesser und Länge) durch biochemische Eingriffe in die Fibrillogenese. Da die Fibrillen im Kompositmaterial die Funktion einer Faserverstärkung einnehmen, hat ihre Geometrie Einfluss auf die mechanischen Eigenschaften des Verbundmaterials.

Mithilfe von Aminozucker-haltigen Polysacchariden, bevorzugt von Glykosaminoglykanen und deren Analoga, lässt sich die Fibrillengeometrie vorteilhaft gezielt beeinflussen. Zu Glykosaminoglykanen analoge Verbindungen sind chemische Verbindungen mit gleicher biologischer Wirkung. Besonders bevorzugt ist dabei die biochemische Modifikation des Kollagens durch die Glykosaminoglykane Chondroitinsulfat, Dermatansulfat, Heparin, Heparansulfat, Hyaluronsäure und Keratansulfat, aber auch durch Proteoglykane, wie Decorin, und andere Aminozucker-haltige Polysaccharide wie Chitosan. [Wolf C., Hanke T., Scharnweber D., Peters K., Kirkpatrick C. J., Worch H.: Influence of artificial extracellular matrices on Ti6Al4V implants on binding and release of VEGF, Abstracts Internationales Symposium ,Interface Biology of Implants', Rostock 14.-16.05.2003, in: BIOmaterialien 4: 158, 2003; Wolf-Brandstetter C., Lode A., Hanke T., Scharnweber D., Worch H.: "Influence of Modified Extracellular Matrices on Ti6Al4V implants on binding and release of VEGF". J. Biomed. Mat. Res A. 79: 882-894, 2006; Abschlußbericht "Innovative Materialsysteme für die Funktionalisierung von Implantaten und den Gewebeaufbau in der Implantologie" (Autoren: Worch, H., Scharnweber, D., Hanke, T., 2002) BMBF-Projekt 03N4015/9 (10/1999 bis 10/2002); Sammlung "Deutsche Forschungsberichte" TIB/UB Hannover].

Die biochemische Modifikation erfolgt durch den Einbau der genannten Substanzen in die Kollagenmatrix. Der Einbau erfolgt bevorzugt entweder durch adsorptive Immobilisierung, oder durch kovalente Quervernetzung, beispielsweise mit N-(3-dimethylaminopropyl)-N'-ethylcarbodiimide (EDC). Der Einbau kann alternativ auch während der spontanen Zusammenlagerung der Fibrillen stattfinden, die bei neutralem pH-Wert (z. B. in Phosphatpuffer oder Tris-Puffer) und auf ca. 37°C erhöhter Temperatur erreicht wird. Im Anschluss daran erfolgt dann gegebenenfalls eine kovalente Quervernetzung.

Die Zusammensetzung enthält 40-92,5 % Silikat, 7,5-60 % Kollagen, bevorzugt 20-60 % Kollagen, und bis zu 52,5 % Calciumphosphat, bevorzugt bis zu 15 % Calciumphosphat.

Als vorteilhaft für die Herstellung von Xerogelen des erfindungsgemäßen Kompositmaterials haben sich Zusammensetzungen von 40-92,5 % Silikat, 7,5-30 % Kollagen und bis zu 30 % Calciumphosphat herausgestellt. Als besonders bevorzugt gilt eine Zusammensetzung von 60-80 % Silikat, 10-20 % Kollagen und 10-20 % Calciumphosphat. Ganz besonders bevorzugt ist eine Zusammensetzung von 65-80 % Silikat, 10-20 % Kollagen und 10-15 % Calciumphosphat.

Für die Herstellung poröser Kompositmaterialien ist der Kollagenanteil auf ca. 50-90 % zu erhöhen.

Bestandteil der Erfindung ist weiterhin ein Verfahren nach Anspruch 8 zur Herstellung eines Kompositmaterials aus einer mit Silikat und Calciumphosphatphasen mineralisierten sowie gegebenenfalls biochemisch modifizierten Kollagenmatrix. Das Verfahren beruht auf dem Sol-Gel-Prozess. Dazu werden Calciumphosphatphasen in einer homogenen Kollagensuspension gelöst. Im zweiten Schritt erfolgt die Silikatbildung durch die Zugabe von Kieselsäure in Form eines hydrolysierten Silizium-Precursors, wobei sich zunächst ein Sol bildet.

Vorteilhaft lässt sich durch das Einbringen von Kollagenfibrillen ganz ohne thermische Nachbehandlung ein monolithisches Material erhalten. Dadurch wird zum einen der Energieaufwand minimiert, andererseits lässt sich die Endstruktur des Materials mithilfe der organischen Komponente gezielt einstellen. Bei einer thermischen Nachbehandlung, bei der die organische Komponente durch die Temperatureinwirkung zersetzt und irreversibel zerstört wird, bestünde diese Möglichkeit hingegen nicht.

Die Kollagenkonzentration ist vorteilhaft so hoch wie möglich zu wählen, wobei die resultierende Suspension nicht zu viskos sein darf, um eine Verarbeitung und spätere Mischbarkeit zu gewährleisten. Die Kollagenkonzentration entspricht 5-40 mg/ml, besonders bevorzugt 20-25 mg/ml. Als Kollagenkomponente des erfindungsgemäßen Kompositmaterials wird Kollagen in Form von Kollagenfibrillen oder von Kollagenfasern eingesetzt.

Als Lösungsmittel der Kollagensuspension können prinzipiell alle Lösungsmittel eingesetzt werden, die das Kollagen nicht in seinen notwendigen Eigenschaften verändern und außerdem bei Zusammenführung mit der Calciumphosphatkomponente und der Silikatkomponente die genannten Mineralisationsprozesse erlauben. Gleiches gilt für die Lösung des Silizium-Precursors.

Bevorzugt wird das Kollagen in einem Lösungsmittel mit neutralem pH-Wert, also vorzugsweise bei pH 6,5 bis 7,5, bevorzugt bei pH 6,8 bis 7,4, besonders bevorzugt bei pH 7,0 bis 7,2, suspendiert.

Vorteilhaft wird dazu zunächst die Calciumphosphatphase in Pulverform der homogenen Kollagensuspension beigemischt. Eine homogene Durchmischung der Komponenten erfolgt bevorzugt mithilfe eines Vortexers, eines Propellerrührers, eines Magnetrührers, eines Statikmischers oder einer Schwingmühle. Alternativ kann das Calciumphosphat zuvor in dem Puffer gelöst werden, in dem dann das Kollagenlyophilisat suspendiert wird. Dabei findet eine Anbindung der Calciumphosphatphase an das Kollagen statt. Als Calciumphosphatphase dienen alle Calciumphosphatphasen, die in Form eines Pulvers in die Kollagensuspension gegeben werden können. In einer besonders bevorzugten Ausführungsform wird als Calciumphosphatphase Hydroxylapatit (Ca₁₀(PO₄)₆(OH)₂), Tricalciumphosphat (Ca₃PO₄), amorphes Calciumphosphat (Caₓ(PO₄)_{y}·nH₂O), Octacalciumphosphat (Ca₈H₂(PO₄)₆·5H₂O) oder Brushit (CaHPO₄·2H₂O) oder Mischungen dieser Calciumphosphatphasen untereinander wie beispielsweise Calciumphosphatzement (eine Mischung aus Ca₃(PO₄)₂, CaCO₃, Ca(HCO₃) und Ca₁₀(PO₄)₆(OH)₂), oder Mischungen von bzw. mit Calcium- und Phosphatsalzen wie beispielsweise CaCl₂ und/oder Na₂HPO₄ genutzt. Die Calciumphosphatphase kann zusätzlich mit Ionen wie Fluorid, Silber, Magnesium oder Carbonat dotiert sein.

Das resultierende Kollagen-Calciumphosphat-Gemisch wird mit der Kieselsäure versetzt und vermischt. Um die für alle Silikatisierungsreaktionen vorausgesetzte Kieselsäure zu synthetisieren, wird erfindungsgemäß ein Silizium-Precursor eingesetzt. In einer bevorzugten Ausführungsform des Kompositmaterials ist der Silizium-Precursor eine hydrolysierte Alkoxysilan-Lösung (Si(OR)₄, R=CₙH₂ₙ₊₁) oder Wasserglas, beispielsweise Na₂SiO₃. In einer besonders bevorzugten Ausführungsform ist die Alkoxysilan-Lösung eine Tetramethoxysilan (TMOS; (Si(OCH₃)₄)-Lösung oder eine Tetraethoxysilan(TEOS; (Si(OC₂H₅)₄)-Lösung, vorzugsweise im molaren Verhältnis TMOS:Wasser bzw. TEOS:Wasser 1:4.

Die Fibrillen in den mit Calciumphosphatphasen angereicherten Kollagensuspensionen dienen als biologisches Templat und erzwingen die gezielte Polymerisation der Kieselsäure bzw. des Silikats an den Kollagenfibrillen. Die Keimbildung beruht auf den attraktiven Wechselwirkungen zwischen den positiv geladenen Aminogruppen des Kollagens und den negativ geladenen Oxidgruppen der Kieselsäure bzw. Silikatpartikel. Das Kollagen selbst beschleunigt konzentrationsabhängig die heterogene Keimbildung der Kieselsäurepolymerisation an den Fibrillen. An den Fibrillen des Kollagens bilden sich nanometergroße Silikatpartikel, agglomerieren dort und silikatisieren diese somit.

Die Kieselsäure vernetzt bei der Polykondensation zu Silikat einzelne Kollagenmoleküle oder -fibrillen untereinander. Bei dieser Quervernetzung findet eine Einlagerung der Calciumphosphatphasen statt. Solange der Gelpunkt noch nicht erreicht ist, lässt sich die Mischung in nahezu beliebige Form bringen und sich somit die endgültige Probengestalt bestimmen. Durch die Polymerisierung der Kieselsäure an den Kollagenfibrillen (Silikatisierung) und die dadurch entstehende Vernetzung der Kollagenfibrillen untereinander bildet sich ein Hydrogel. In Abhängigkeit von den stofflichen Anteilen des Silikats, Kollagens und Calciumphosphats lassen sich so Hydrogele erzeugen.

Um ein homogenes Hydrogel mit definierter Zusammensetzung zu erhalten, sollte die Gelbildung abgeschlossen sein, bevor sich die Suspension entmischen kann. Um zu gewährleisten, dass die Gelbildung rasch statt findet, wird das Kollagen bevorzugt in einem Lösungsmittel mit neutralem pH-Wert suspendiert.

Der Gelbildungsprozess läuft dann im Allgemeinen innerhalb weniger Sekunden ab. In dieser Zeit muss eine homogene Intensivvermischung der Komponenten erreicht werden. Dies erreicht man bevorzugt mithilfe eines Vortexers, eines Propellerrührers, eines Magnetrührers, eines Statikmischers oder einer Schwingmühle. Falls dies verfahrenstechnisch notwendig sein sollte, lässt sich die Verarbeitungszeit, d. h. die Zeit bis zur Gelbildung, verlängern, indem die Ausgangskomponenten auf niedrige Temperaturen (0-15°C) gekühlt werden. Auch durch eine geringfügige Erniedrigung des pH-Werts, bevorzugt auf pH 6,0 bis pH 6,8, besonders bevorzugt pH 6,4 bis 6,5, kann die Verarbeitungszeit verlängert werden. Im Anschluss daran wird nach erfolgter Gelbildung mit neutralem Puffer gespült, um wieder einen neutralen pH-Wert des Materials zu erreichen.

Die erhaltenen Hydrogele lassen sich durch Trocknung, bevorzugt unter besonderen klimatischen Bedingungen, in monolithische Xerogele aus dem erfindungsgemäßen dreikomponentigen Kompositmaterial überführen.

Bestandteil der Erfindung ist daher auch ein Verfahren zur Herstellung eines Hydrogels aus einer mit Silikat und Calciumphosphatphasen mineralisierten Kollagenmatrix, bei dem zunächst die Calciumphosphatphasen in einer homogenen Kollagensuspension gelöst und die Kollagenfasern in einem zweiten Schritt durch die Zugabe von Kieselsäure silikatisiert werden, und bei dem das Produkt anschließend nach Erreichen des Gelpunkts zur Festigung der Festkörperstruktur unter Luftabschluss gelagert wird.

Die erfindungsgemäßen Hydrogele können durch Trocknen, vorzugsweise durch Trocknen an der Luft, in ein Xerogel überführt werden. Bestandteil der Erfindung ist daher auch ein Verfahren, bei dem zur Herstellung eines Xerogels ein erfindungsgemäßes Hydrogel getrocknet, bevorzugt luftgetrocknet, wird.

Vorteilhaft werden die Hydrogele dazu zunächst mindestens 24 Stunden unter Luftabschluss ausgelagert. Dabei stabilisiert sich das Gelnetzwerk.

Die anschließende Trocknung kann bei Raumklima oder in einem Trockenschrank durchgeführt werden. Bevorzugt wird die Trocknung möglichst langsam vollzogen, um die Gelstabilität nicht zu überschreiten. Vorzugsweise werden dazu die Gele zunächst bei maximal 40 °C, bevorzugt bei 37°C, und bei maximal 100 % relativer Feuchte, bevorzugt bei 95 % relativer Feuchte, gehalten und sukzessive auf Raumklima, das ist bevorzugt eine Temperatur von 20 bis 25 °C und eine relative Feuchte von 20-60 %, gefahren. Die Geschwindigkeit, mit der die Temperatur und die relative Feuchte während des Trocknungsvorgangs verändert werden, hängt von der Größe des zu trocknenden Hydrogels ab. Geeignete Trocknungsbedingungen lassen sich durch den Fachmann durch einfache Versuche leicht und schnell ermitteln.

Auf diese Weise können monolithische Xerogele erzeugt werden. Bestandteil der Erfindung sind auch die aus dem erfindungsgemäßen Kompositmaterial erhaltenen Xerogele.

Alternativ kann das aus dem erfindungsgemäßen Kompositmaterial erhaltene Hydrogel auch in ein Aerogel überführt werden. Dazu wird die Flüssigphase so aus dem Hydrogel entfernt, dass keine (bzw. nur sehr geringe) Kapillarkräfte auftreten und somit eine Schrumpfung des Materials vermieden wird. Die anzuwendende Trocknung unter überkritischen Bedingungen erfordert zunächst eine Substitution der Flüssigphase (z.B. Wasser und Methanol bei Verwendung von TMOS als Silizium-Precursor) der Hydrogele. Dazu werden die Hydrogele z. B. mehrere Tage in Reinstethanol eingelegt. Das Ethanol wird dabei regelmäßig in Abständen gewechselt. Danach wird die Kritisch-Punkt-Trocknung in einem Autoklaven durchgeführt. Dabei wird das Ethanol zunächst bei einer Temperatur von etwa 7 °C unter Hochdruckbedingungen durch mehrmaliges Spülen durch flüssiges Kohlendioxid ersetzt. Anschließend wird die Temperatur auf 38 bis 40 °C erhöht, wobei das Kohlendioxid in den überkritischen Zustand übergeht, d. h. es existiert keine Grenzfläche mehr zwischen der flüssigen und gasförmigen Phase. Durch ein Ventil wird das überkritische Kohlendioxid bei Aufrechterhaltung der Bedingungen langsam abgeblasen. Durch das Vermeiden der Grenzfläche können keine Kapillarkräfte entstehen und die Festkörperstruktur der Probe bleibt unverändert erhalten. Nach Abschluss dieses Verfahrens werden getrocknete Aerogele erhalten, deren innere und äußere Strukturen denen der eingesetzten Hydrogele entsprechen.

Bestandteil der Erfindung ist daher auch ein Aerogel, das durch Substitution der Flüssigphase eines erfindungsgemäßen Hydrogels und anschließende Trocknung unter überkritischen Bedingungen erhältlich ist.

Bestandteil der Erfindung ist weiterhin ein Verfahren zur Herstellung eines Aerogels, bei dem die Flüssigphase eines erfindungsgemäßen Hydrogels substituiert und im Anschluss eine Trocknung unter überkritischen Bedingungen durchgeführt wird.

Anstelle von Gelen sind ausgehend von dem erfindungsgemäßen Verfahren durch Gefriertrocknung auch mit Silikat und Calciumphosphat mineralisierte poröse Kollagenscaffolds herstellbar. Dazu wird zunächst eine Calciumphosphatphase in einer homogenen Kollagensuspension gelöst. Je nach verwendetem Kollagen kann die Kollagenkonzentration dabei so weit gesteigert werden, wie sich die erhaltene Suspension noch homogen mit der Silikat-Komponente (pre-hydrolysierten Alkoxysilan-Lösung) vermischen lässt. Um den Charakter eines porösen Kollagenscaffolds - und nicht den eines Silikatgels - zu erhalten, werden vorteilhaft Kollagenanteile von ca. 50-90 % bevorzugt.

Bestandteil der Erfindung ist daher auch ein Verfahren, bei dem zur Herstellung eines mit Silikat und einer Calciumphosphatphase mineralisierten porösen Kollagenscaffolds zunächst eine Calciumphosphatphase in einer homogenen Kollagensuspension gelöst wird, und das Produkt mit einer geringen Menge einer hydrolysierten Alkoxysilan-Lösung gemischt und anschließend eine Gefriertrocknung durchgeführt wird.

Bestandteil der Erfindung ist daher auch ein mit Silikat und einer Calciumphosphatphase mineralisiertes poröses Kollagenscaffold, das durch Gefriertrocknung eines erfindungsgemäßen Kompositmaterials erhältlich ist.

Weiterhin eignet sich das erfindungsgemäße Verfahren zur Herstellung von Kompositpartikeln. Kompositpartikel sind erhältlich, indem eine mit Calciumphosphat angereicherte homogene Kollagensuspension zu einer Alkoxysilan-Lösung gegeben wird. Zur Herstellung sphärischer Partikel wird zunächst die Calciumphosphatphase in einer homogenen Kollagensuspension gelöst und die entsprechend konzentrierte Lösung tropfenweise in eine reine Alkoxysilan-Lösung pipettiert. Beim Eintropfen der mit einer Calciumphosphatphase versetzten Kollagensuspension neutralen pH-Werts (z. B. Tris/HCl-Puffer) in die Alkoxysilan-Lösung führt eine lokale Hydrolyse durch das Wasserangebot und die Templatfunktion des Kollagens zur spontanen Polymerisation der Kieselsäure mit dem Ergebnis eines kugelförmigen Kompositpartikels. Diese Kugel wird nach ca. 24 Stunden aus der Lösung genommen, gespült und luftgetrocknet. Das resultierende Produkt entspricht somit einem sphärisch geformten Komposit-Xerogel.

Bestandteile der Erfindung sind daher ein Verfahren, bei dem zur Herstellung von Partikeln zunächst die Calciumphosphatphase in einer homogenen Kollagensuspension gelöst bzw. suspendiert und die Lösung anschließend zu einer Alkoxysilan-Lösung gegeben wird, sowie Partikel aus dem erfindungsgemäßen Kompositmaterial.

Als Partikel ist das erfindungsgemäße Kompositmaterial z. B. als Wirkstoffträger ("Drug delivery system") geeignet. Bestandteil der Erfindung ist daher auch die Verwendung des Partikels als Wirkstoffträger.

Das erfindungsgemäße Kompositmaterial eignet sich außerdem zur Beschichtung eines geeigneten Substrats, beispielsweise Ti6Al4V. Zur Herstellung einer solchen Beschichtung wird zunächst in einer homogenen Kollagensuspension eine Calciumphosphatphase gelöst bzw. suspendiert und die Lösung anschließend mit einer Alkoxysilan-Lösung gemischt. Die Konzentrationen der Kollagensuspension und der Alkoxysilan-Lösung werden dabei so niedrig gewählt, dass die Mischlösung den Gel-Punkt im Versuchszeitraum nicht erreicht. Zur Beschichtung werden die Substrate (z. B. biokompatible metallische Platten) in die Mischlösung getaucht und langsam herausgezogen. Dabei scheidet sich eine Schicht auf den Substraten ab, deren Dicke und Beschaffenheit vor allem von der Zusammensetzung der Mischlösung und der Ziehgeschwindigkeit abhängen. An Luft trocknen die Schichten zu einem dichten und mechanisch stabilen Substratüberzug

Bestandteil der Erfindung ist daher auch ein Beschichtungsmaterial aus einem erfindungsgemäßen Kompositmaterial sowie ein Verfahren, bei dem zur Beschichtung eines Substrats mit einem Kompositmaterial zunächst eine Calciumphosphatphase in einer homogenen Kollagensuspension gelöst bzw. suspendiert wird, die Lösung mit einem Silizium-Precursor gemischt wird und das Substrat in die Mischlösung getaucht und anschließend getrocknet wird.

Bestandteil der Erfindung ist auch die Verwendung dieser Beschichtung zur Biologisierung von Implantatoberflächen. Unter biologisierender Beschichtung versteht man die Veredelung eines Substrates mit einer biologisch wirksamen und/oder bioaktiven Substanz.

Die Erfindung beruht auf der überraschenden wissenschaftlichen Erkenntnis, dass im Dreistoffsystem Kollagen-Silikat-Calciumphosphat unter definierten Bedingungen makroskopische Kompositmaterialien herstellbar sind, deren biologische und mechanische Eigenschaften in einem weiten Bereich variiert werden können. Das erlaubt eine gezielte Anpassung der Materialeigenschaften an den Einsatzort, wie es bisher nicht möglich war.

Das erfindungsgemäße Kompositmaterial aus einer mit Silikat und Calciumphosphat mineralisierten Kollagenmatrix ist biokompatibel, bioaktiv und hält auch größeren mechanischen Belastungen stand.

Durch die biochemische Modifikation der Fibrillengeometrie können gezielt die mechanischen Eigenschaften des erfindungsgemäßen Kompositmaterials beeinflusst werden. Durch die biochemische Modifikation durch Aminozucker-haltige Polysaccharide wie beispielsweise Glykosaminoglykane, z. B. Chondroitinsulfat, Dermatansulfat, Heparin, Heparansulfat, oder Chitosan, und/oder Proteoglykane (Decorin), kann die Geometrie der Kollagenfibrillen gezielt beeinflusst werden. Bestandteil der Erfindung ist daher auch ein Verfahren, bei dem das Kollagen vor der Mischung mit Calciumphosphatphasen und der Silikatisierung durch den Silizium-Precursor zunächst durch Aminozucker-haltige Polysaccharide, bevorzugt Glykosaminoglykane, modifiziert wird.

Das erfindungsgemäße Kompositmaterial aus einer zweifach mineralisierten sowie gegebenenfalls biochemisch modifizierten Kollagenmatrix ist außerdem strukturell und stofflich in weiten Grenzen variierbar und somit zur Herstellung vielfältigster Applikationen mit mechanisch und biologisch angepassten Eigenschaften einsetzbar. Durch die Nutzung der Sol-Gel-Technik und aufgrund der mechanischen Bearbeitbarkeit kann das Material in verschiedenen geometrischen Formen hergestellt werden.

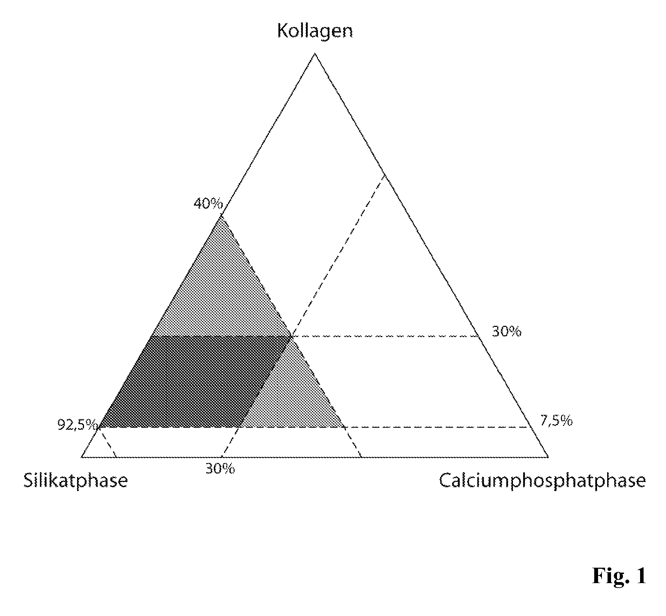

Die stoffliche Zusammensetzung kann im gesamten Dreistoffsystem variiert werden (siehe Fig. 1). Bevorzugt ist eine Zusammensetzung von 40-92,5 % Silikat, 7,5-60 % Kollagen und bis zu 52,5 % Calciumphosphat (Fig. 1, grau markierte Bereiche). Als vorteilhaft für die Herstellung von Xerogelen des erfindungsgemäßen Kompositmaterials haben sich Zusammensetzungen von 40-92,5 % Silikat, 7,5-30 % Kollagen und Calciumphosphat herausgestellt (Fig. 1, dunkelgrau markierter Bereich).

Die erfindungsgemäßen Kompositmaterialien sind prinzipiell für technische und biologische Zwecke einsetzbar. Sie können als Konstruktionswerkstoff und/oder als Funktionswerkstoff verwendet werden. Sie sind biomedizinisch als Implantatmaterial einsetzbar, bei dem neben guter Biokompatibilität eine hinreichende Festigkeit (z. B. im Knochenkontakt) gefordert wird. Sie finden bevorzugt Verwendung als Implantatmaterial im unbelasteten aber auch lasttragenden Bereich. Die Anwendung reichen von der einfachen Defektfüllung (Kiefer-, Schädelbereich) bis zur Funktionsübernahme des Materials z. B. als Stift zur Fixierung von Brüchen oder Bändern. Durch die vorteilhafte Beeinflussung der mechanischen Eigenschaften der erfindungsgemäßen Kompositmaterialien sowie ihre hohe Bioaktivität eignen sie sich für eine hohe Zahl von Anwendungsmöglichkeiten. Sie lassen sich zu Formstücken modellieren, können aber auch zur biologisierenden Beschichtung von Implantaten aus anderen Materialien einsetzen. Dazu wird das zu beschichtende Substrat in das Kompositmaterial getaucht, solange der Gelpunkt noch nicht erreicht wurde, und anschließend getrocknet.

Eine weitere Verwendungsmöglichkeit besteht in der "Beladung" der Kompositmaterialien mit pharmazeutischen Wirkstoffen. Dies kann einerseits zur Herstellung freisetzungsverzögernder Verabreichungssysteme verwendet werden. Alternativ lassen sich Implantate beispielsweise mit bioaktiven Wirkstoffen (Faktoren) beladen, die gezielt nützliche Zellreaktionen auslösen. Gerade im Knochenkontakt kann dadurch das Verhältnis der Osteoklasten-Osteoblastentätigkeit beeinflusst werden. Bevorzugt werden dazu Bisphosphonate verwendet.

Anhand nachfolgender Figuren und Ausführungsbeispiele wird die Erfindung näher erläutert, ohne diese einzuschränken.
Figur 1 zeigt bevorzugte Zusammensetzungen des erfindungsgemäßen Dreistoffsystems.
Figur 2 zeigt die so erhaltenen scheibenförmigen (Fig. 2 a) und zylindrischen (Fig. 2 b) Xerogele.
Figur 3 zeigt Rasterelektronenmikroskop-Aufnahmen von Kompositmaterialien aus 100 % Silikat (a, b); 85 % Silikat, 15 % Kollagen (c, d); 70 % Silikat, 30 % Kollagen (e, f); 70 % Silikat, 15 % Kollagen, 15 % Calciumphosphatzement (g, h) in zwei verschiedenen Auflösungen, wobei der eingeblendete weiße Balken jeweils einer Länge von 100 µm (Fig. 3. a, c, e und g) bzw. 10 µm (Fig. 3. b, d, f und h) entspricht.
Figur 4 zeigt die Abhängigkeit der Druckfestigkeit des Kompositmaterials in Abhängigkeit von der verwendeten Calciumphosphatphase.

### Ausführungsbeispiel 1

### Zylindrische Proben des Kompositmaterials aus Silikat, Kalbshautkollagen und Hydroxylapatit.

Lyophilisat von Kalbshautkollagen wird in neutralem Tris/HCl-Puffer (100 mM, pH 7,4) über 24 Stunden unter Rühren suspendiert, so dass sich eine Konzentration von 20 mg/ml ergibt. Hydroxylapatit (Ca₁₀(PO₄)₆(OH)₂) wird in Pulverform in die Kollagensuspension gegeben und über 24 Stunden unter Rühren gelöst. Alternativ kann das Calciumphosphat zuvor in dem Puffer gelöst werden, in dem später das Kollagenlyophilisat suspendiert wird. Die einzuwiegende Menge an Calciumphosphatphasen sowie die entsprechenden Volumina an Kieselsäure und Kollagensuspension richten sich nach der angestrebten Zusammensetzung. In diesem Ausführungsbeispiel wird eine Masse-Zusammensetzung von 70 % Silikat, 15 % Kollagen und 15 % Calciumphosphat angeführt.

Zur Erzeugung von Kieselsäure wird Tetraethoxysilan (TEOS, 99 % Sigma) durch Zugabe von Wasser und Salzsäure (10 mM) als Katalysator 24h bei 4°C hydrolysiert. Das molare Verhältnis von Tetraethoxysilan zu Wasser beträgt 1:4. Diese Lösung dient als Silikatkomponente und wird der mit Calciumphosphat angereicherten Kollagensuspension zugeführt und intensiv vermischt. Nach wenigen Minuten bildet sich ein Hydrogel.

Das eingesetzte Volumen pro Probe und die Form, in die die Mischungen gegeben werden bestimmen die schlussendlich erhaltene Geometrie des Xerogels. Ein Volumen von 500 µl in zylindrischen Gefäßen von 14 mm Durchmesser resultiert in scheibenförmigen Xerogelen von 5 mm Durchmesser und 2 mm Höhe. Ein Volumen von 3500 µl bei 15 mm Durchmesser resultiert in zylindrischen Xerogelen mit etwa 11 mm Durchmesser und 16 mm Höhe.

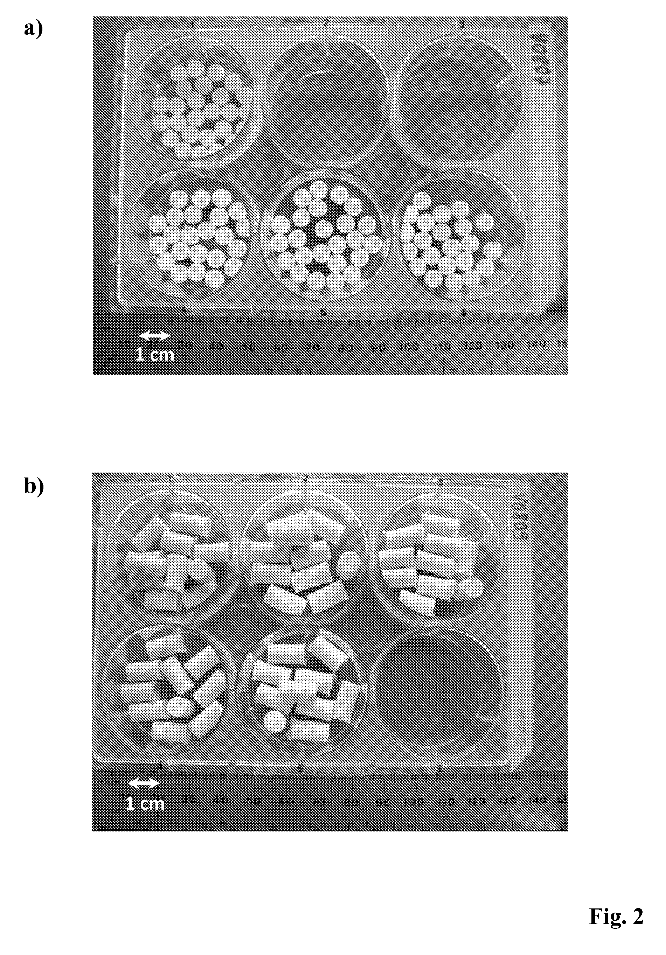

Die Hydrogele werden unter Luftabschluss für 24 Stunden bei Raumtemperatur stabilisiert. Die Trocknung zu Xerogelen erfolgt bei geöffneten Gefäßen bei 37°C und 95 % relative Feuchte. Nach 7 Tagen wird das Klima kontinuierlich über 24 Stunden auf 20°C und 30 % relative Feuchte reguliert. **Figur 2** zeigt die für eine Zusammensetzung von 85 % Silikat, 15 % Kollagen bzw. 70 % Silikat, 15 % Kollagen, 15 % Calciumphosphat erhaltenen scheibenförmigen **(****Fig. 2 a****,** obere Reihe: 85 % Silikat, 15 % Kollagen; untere Reihe Dreistoffsysteme 70 % Silikat, 15 % Kollagen und 15 % Calciumphosphat, von links nach rechts: Calciumphosphatzement, Hydroxylapatit, amorphes Calciumphosphat) und zylindrischen **(****Fig. 2 b****,** obere Reihe links: 85 % Silikat, 15 % Kollagen, mittig: Dreistoffsystem mit Calciumphosphatzement, rechts: Dreistoffsystem mit Hydroxylapatit; untere Reihe Dreistoffsysteme mit rechts: amorphes Calciumphosphat, mittig: Salzmischung) Xerogele. Die zylindrischen Xerogele werden auf einer konventionellen Drehmaschine spanabhebend bearbeitet.

### Ausführungsbeispiel 2

### Charakterisierung der Bioaktivität der Xerogele

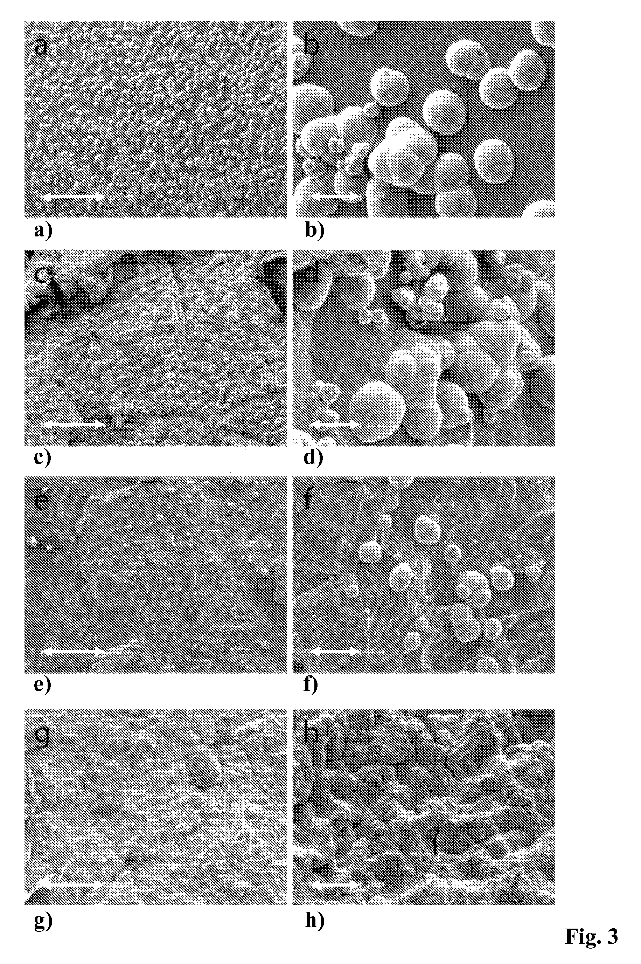

Die wie in Ausführungsbeispiel 1 beschrieben erhaltenen scheibenförmigen Xerogele des Kompositmaterials werden in simulierter Körperflüssigkeit (SBF) ausgelagert. Nach 7 Tagen werden die Proben entnommen und mittels Rasterelektronenmikroskopie untersucht. **Fig. 3** zeigt Aufnahmen von Kompositmaterialien aus 100 % Silikat **(****Fig. 3 a, b****);** 85 % Silikat, 15 % Kollagen **(****Fig. 3 c, d****);** 70 % Silikat, 30 % Kollagen **(****Fig.** 3e, **f**); 70 % Silikat, 15 % Kollagen, 15 % Calciumphosphatzement **(****Fig. 3 g, h****).**

Der eingeblendete Maßstabsbalken entspricht jeweils einer Länge von 100 µm **(****Fig. 3****. a, c, e** und g). bzw. 10 µm **(****Fig. 3****. b, d, f und h).**

Der *in vitro* gebildete Hydroxylapatit ist inselförmig auf der Oberfläche zu erkennen. Die Aufnahmen zeigen, dass ausgehend vom reinen Silikat die Bioaktivität mit zunehmendem Kollagenanteil leicht abnimmt, d. h. die Dichte der gebildeten Hydroxylapatitinseln nimmt ab. Für das erfindungsgemäße Dreistoffsystem mit Calciumphosphatzement **(****Fig. 3g, h****)** ist hingegen eine stark erhöhte *in vitro*-Bioaktivität festzustellen, d. h. es hat sich eine geschlossene Apatitschicht gebildet, die die gesamte mineralisierte Kollagenmatrix überzieht. Die höhere Auflösung **(****Fig. 3 h)** zeigt, dass die Apatitkristalle kleiner sind, als in den Vergleichsbeispielen, die kein Calciumphosphatzement enthalten **(****Fig. 3b, d und f****).**

### Ausführungsbeispiel 3:

### Vergleich verschiedener Calciumphosphatphasen im Kompositmaterial

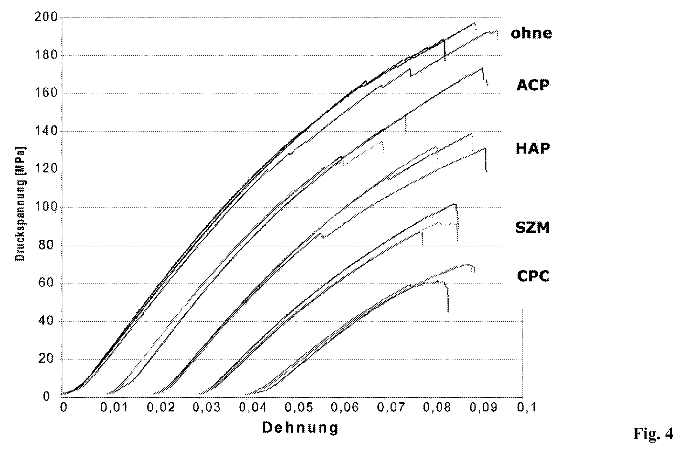

Die Probenherstellung wird analog zu Ausführungsbeispiel 1 durchgeführt. Als Calciumphosphatphase wird neben Hydroxylapatit (HAP) auch Calciumphosphatzement (CPC; eine Mischung aus Ca₃(PO₄)₂, CaCO₃, Ca(HCO₃) und Ca₁₀(PO₄)₆(OH)₂), amorphes Calciumphosphat (ACP; Caₓ(PO₄)y·nH₂O) und eine Salzmischung (SZM, bestehend aus CaCl₂, Na₂HPO₄) eingesetzt. Als Vergleichsprobe dient das Zweikomponentensystem aus 85 % Silikat und 15 % Kollagen. Es werden zylindrische Proben hergestellt, welche mittels Druckversuch auf ihre mechanischen Eigenschaften getestet werden. **Fig. 4** zeigt, wie sich die Druckfestigkeit des Kompositmaterials in Abhängigkeit von der verwendeten Calciumphosphatphase verändert. Auf diese Weise können die Eigenschaften des Kompositmaterials an den Einsatzort angepasst werden.

## Patentansprüche

1. Monolithisches Kompositmaterial umfassend eine mit Silikat und einer Calciumphosphatphase mineralisierte Kollagenmatrix, wobei das Kollagen rekombinantes Kollagen, Kollagen aus Eumetazoa, Schwammkollagen der Klassen Demospongia oder Calcarea oder eine Mischung dieser Kollagene ist, wobei die Zusammensetzung der Komponenten 40 - 92,5 % Silikat, 7,5 - 60 % Kollagen und bis zu 52,5 % Calciumphosphat beträgt und wobei das Kollagen in Form von Kollagenfibrillen oder Kollagenfasern vorliegt.

2. Kompositmaterial nach Anspruch 1, **dadurch gekennzeichnet, dass** die Calciumphosphatphase ausgewählt ist aus der Gruppe bestehend aus Calciumphosphatzementmischung aus Ca₃(PO₄)₂, CaCO₃, Ca(HCO₃) und Ca₁₀(PO₄)₆(OH)₂), Hydroxylapatit, Tricalciumphosphat, amorphem Calciumphosphat, Octacalciumphosphat, Brushit, Mischungen dieser Calciumphosphatphasen untereinander und Mischungen von Calcium- und Phosphatsalzen.

3. Kompositmaterial nach einem der Ansprüche 1 oder 2, **dadurch gekennzeichnet, dass** das Kollagen durch ein Aminozucker-haltiges Polysaccharid und/oder ein Proteoglykan biochemisch modifiziert ist.

4. Kompositmaterial nach einem der Ansprüche 1 bis 3, **dadurch gekennzeichnet, dass** es einen pharmazeutischen Wirkstoff enthält.

5. Hydrogel, erhältlich durch Lagern eines Kompositmaterials nach einem der Ansprüche 1 bis 4.

6. Monolithisches Xerogel, erhältlich durch Trocknen eines Hydrogels nach Anspruch 5, wobei die Trocknung bei Raumklima oder in einem Trockenschrank durchgeführt wird, wobei die Gele zunächst bei maximal 40 °C, bevorzugt bei 37°C, und bei maximal 100 % relativer Feuchte, bevorzugt bei 95 % relativer Feuchte, gehalten und sukzessive auf Raumklima, bevorzugt eine Temperatur von 20 bis 25 °C und eine relative Feuchte von 20-60 %, gefahren werden.

7. Beschichtungsmaterial oder Partikel oder poröse Schaffolds oder Aerogel aus einem Kompositmaterial nach einem der Ansprüche 1 bis 4.

8. Verfahren zur Herstellung eines monolithischen Kompositmaterials umfassend eine mit Silikat und einer Calciumphosphatphase mineralisierte Kollagenmatrix, umfassend die folgenden Verfahrensschritte:
a) Lösen einer Calciumphosphatphase in einer homogenen Kollagensuspension, wobei die Kollagenkonzentration 5-40 mg/ml beträgt und das Kollagen in Form von Kollagenfibrillen oder Kollagenfasern vorliegt,
b) Zugabe von Kieselsäure in Form eines Silizium-Precursors,
wobei die Zusammensetzung der Komponenten 40 - 92,5 % Silikat, 7,5 - 60 % Kollagen und bis zu 52,5 % Calciumphosphat beträgt.

9. Verfahren nach Anspruch 8, **dadurch gekennzeichnet, dass** zur Herstellung eines Hydrogels eine Calciumphosphatphase in einer homogenen Kollagensuspension gelöst und anschließend mit einer hydrolysierten Alkoxysilan-Lösung gemischt wird und das Produkt nach Erreichen des Gelpunkts zur Festigung der Festkörperstruktur in einem geeigneten Lösungsmittel unter Luftabschluss gelagert wird.

10. Verfahren nach Anspruch 9,
**dadurch gekennzeichnet, dass** zur Herstellung eines Xerogels das Hydrogel getrocknet wird, wobei die Trocknung bei Raumklima oder in einem Trockenschrank durchgeführt wird , wobei die Gele zunächst bei maximal 40 °C, bevorzugt bei 37°C, und bei maximal 100 % relativer Feuchte, bevorzugt bei 95 % relativer Feuchte, gehalten und sukzessive auf Raumklima, bevorzugt eine Temperatur von 20 bis 25 °C und eine relative Feuchte von 20-60 %, gefahren werden.

11. Verfahren nach Anspruch 9,
**dadurch gekennzeichnet, dass** zur Herstellung eines Aerogels die Flüssigphase des Hydrogels substituiert wird und im Anschluss eine Trocknung unter überkritischen Bedingungen durchgeführt wird.

12. Monolithisches Kompositmaterial nach einem der Ansprüche 1 bis 5 zur Verwendung als Konstruktionswerkstoff und/oder Funktionswerkstoff oder als Implantatmaterial oder zur biologisierenden Beschichtung eines Substrats oder als Wirkstoffträger.

13. Knochenersatzmaterial enthaltend ein Kompositmaterial nach einem der Ansprüche 1 bis 5, ein Hydrogel nach Anspruch 5, ein Xerogel nach Anspruch 6 oder ein Aerogel nach Anspruch 7.

## Claims

1. A monolithic composite material comprising a collagen matrix which is mineralized with silicate and a calcium phosphate phase, wherein the collagen is recombinant collagen, collagen from Eumetozoa, sponge collagen from the Demospongia or Calcarea classes or a mixture of said collagens, wherein the component composition is 40% - 92.5% of silicate, 7.5% - 60% of collagen, and up to 52.5% of calcium phosphate and wherein the collagen is in the form of collagen fibrils or collagen fibres.

2. The composite material as claimed in claim 1, **characterized in that** the calcium phosphate phase is selected from the group consisting of a calcium phosphate cement mixture of Ca₃(PO₄)₂, CaCO₃, Ca(HCO₃) and Ca₁₀(PO₄)₆(OH)₂, hydroxyapatite, tricalcium phosphate, amorphous calcium phosphate, octacalcium phosphate, brushite, mixtures of said calcium phosphate phases and mixtures of calcium salts and phosphate salts.

3. The composite material as claimed in claim 1 or claim 2, **characterized in that** the collagen has been biochemically modified with an amino sugar-containing polysaccharide and/or a proteoglycan.

4. The composite material as claimed in one of claims 1 to 3, **characterized in that** it contains a pharmaceutically active ingredient.

5. A hydrogel which can be obtained by storing a composite material as claimed in one of claims 1 to 4.

6. A monolithic xerogel which can be obtained by drying a hydrogel as claimed in claim 5, wherein drying is carried out at ambient atmosphere or in a drying cabinet, wherein the gels are initially obtained at a maximum of 40°C, preferably 37°C, and at a maximum of 100% relative humidity, preferably 95% relative humidity and are gradually brought to ambient atmosphere, preferably a temperature of 20°C to 25°C and to a relative humidity of 20% - 60%.

7. A coating material or particle or porous scaffold or aerogel formed from a composite material as claimed in one of claims 1 to 4.

8. A method for producing a monolithic composite material comprising a collagen matrix mineralized with silicate and a calcium phosphate phase, the method comprising the following steps:
a) dissolving a calcium phosphate phase in a homogeneous collagen suspension, wherein the collagen concentration is 5 - 40 mg/mL and the collagen is in the form of collagen fibrils or collagen fibres;
b) adding silicic acid in the form of a silicon precursor,
wherein the component composition is 40% - 92.5% of silicate, 7.5% - 60% of collagen, and up to 52.5% of calcium phosphate.

9. The method as claimed in claim 8, **characterized in that** in order to produce a hydrogel, a calcium phosphate phase is dissolved in a homogeneous collagen suspension and subsequently mixed with a hydrolysed alkoxysilane solution and after reaching the gel point, in order to strengthen the solid body structure, the product is stored in a suitable solvent with the exclusion of air.

10. The method as claimed in claim 9,
**characterized in that** in order to produce a xerogel, the hydrogel is dried, wherein drying is carried out at ambient atmosphere or in a drying cabinet, wherein the gels are initially obtained at a maximum of 40°C, preferably 37°C, and at a maximum of 100% relative humidity, preferably at 95% relative humidity and are gradually brought to ambient atmosphere, preferably to a temperature of 20°C to 25°C and a relative humidity of 20% - 60%.

11. The method as claimed in claim 9,
**characterized in that** in order to produce an aerogel, the liquid phase of the hydrogel is substituted and subsequently, drying is carried out under supercritical conditions.

12. A monolithic composite material as claimed in one of claims 1 to 5, for use as a construction material and/or a functional material or as an implant material or for coating a substrate for biological purposes or as a substance support.

13. Bone replacement material containing a composite material as claimed in one of claims 1 to 5, a hydrogel as claimed in claim 5, a xerogel as claimed in claim 6 or an aerogel as claimed in claim 7.

## Revendications

1. Matériau composite monolithique comprenant une matrice de collagène minéralisée avec du silicate et une phase phosphate de calcium, dans lequel le collagène est du collagène recombinant, du collagène eumétazoaire, du collagène d'éponge des classes démosponge ou calcaréa ou un mélange de ces collagènes, dans lequel la composition des composants est de 40 à 92,5 % de silicate, 7,5 à 60 % de collagène et jusqu'à 52,5 % de phosphate de calcium et dans lequel le collagène est présent sous forme de fibrilles de collagène ou de fibres de collagène.

2. Matériau composite selon la revendication 1, **caractérisé en ce que** la phase phosphate de calcium est sélectionnée dans le groupe composé de mélange de ciment de phosphate de calcium à base de Ca₃(PO₄)₂, CaCO₃, Ca(HCO₃) et Ca₁₀(PO₄)₆(OH)₂), hydroxylapatite, triphosphate de calcium, phosphate de calcium amorphe, octaphosphate de calcium, brushite, mélanges de ces phases phosphate de calcium entre elles et mélanges de sels de calcium et de phosphate.

3. Matériau composite selon une des revendications 1 ou 2, **caractérisé en ce que** le collagène est modifié biochimiquement par un polysaccharide contenant du sucre aminé et/ou un protéoglycane.

4. Matériau composite selon une des revendications 1 à 3, **caractérisé en ce qu'**il contient un principe actif pharmaceutique.

5. Hydrogel, pouvant être obtenu par stockage d'un matériau composite selon une des revendications 1 à 4.

6. Xérogel monolithique, pouvant être obtenu par séchage d'un hydrogel selon la revendication 5, dans lequel le séchage est réalisé sous atmosphère ambiante ou dans une armoire de séchage, dans lequel les gels sont d'abord maintenus au maximum à 40°C, de préférence à 37 °C, et sous une hygrométrie relative maximale de 100 %, de préférence à une hygrométrie relative de 95 % et sont amenés progressivement au niveau de l'atmosphère ambiante, de préférence à une température de 20 à 25 °C et une hygrométrie relative de 20 à 60 %.

7. Matériau de revêtement ou particules ou scaffolds poreux ou aérogel composés d'un matériau composite selon une des revendications 1 à 4.

8. Procédé de fabrication d'un matériau composite monolithique comprenant une matrice de collagène minéralisée avec du silicate et une phase phosphate de calcium, comprenant les étapes opératoires suivantes :
a) dissolution d'une phase phosphate de calcium dans une suspension homogène de collagène, la concentration de collagène étant de 5 à 40 mg/ml et le collagène étant présent sous forme de fibrilles de collagène ou de fibres de collagène,
b) ajout d'acide salicylique sous forme d'un précurseur de silicium,
dans lequel la composition des composants est de 40 à 92,5% de silicate, 7,5 à 60 % de collagène et jusqu'à 52,5 % de phosphate de calcium.

9. Procédé selon la revendication 8, **caractérisé en ce que**, pour fabriquer un hydrogel, une phase phosphate de calcium est dissoute dans une suspension homogène de collagène puis mélangée à une solution d'alcoxysilane hydrolysée et le produit est stocké sous extrusion d'air après atteinte du point de gélification dans un solvant approprié pour consolider la structure du corps solide.

10. Procédé selon la revendication 9, **caractérisé en ce que**, pour fabriquer un xérogel, l'hydrogels est séché, le séchage étant réalisé sous atmosphère ambiante ou dans une armoire de séchage, les gels étant d'abord maintenus au maximum à 40 °C, de préférence à 37°C, et sous une hygrométrie relative maximale de 100 %, de préférence à une hygrométrie relative de 95 %, et amenés progressivement au niveau de l'atmosphère ambiante, de préférence à une température de 20 à 25 °C et une hygrométrie relative de 20 à 60 %.

11. Procédé selon la revendication 9, **caractérisé en ce que** pour fabriquer un aérogel, la phase liquide de l'hydrogel est substituée puis un séchage est réalisé dans des conditions plus que critiques.

12. Matériau composite monolithique selon une des revendications 1 à 5, destiné à une utilisation comme matériau structurel et/ou matériau fonctionnel ou comme matériau d'implant ou pour le revêtement biologisant d'un substrat ou comme support de principe actif.

13. Matériau de substitution osseuse contenant un matériau composite selon une des revendications 1 à 5, un hydrogel selon la revendication 5, un xérogel selon la revendication 6 ou un aérogel selon la revendication 7.
